# EUROPEAN PATENT APPLICATION

(11) **EP 4 563 094 A1**
(43) Date of publication of application: **04.06.2025**
(21) Application number: 23212810.8
(22) Date of filing: 28.11.2023
(51) Int. Cl.: A61B 10/00, A61B 10/04

(54) **INGESTIBLE DEVICE AND METHOD FOR SAMPLING MATERIAL USING A HEAT DEPENDENT ACTUATING MECHANISM**

(71) Applicant: Stichting IMEC Nederland, 6708 WH Wageningen (NL)
(72) Inventor: Schoeman, Rogier, 3001 Leuven (BE)
(74) Representative: Patent Department IMEC

(57) **Abstract**

An ingestible device (10) for sampling material at least one time is provided. The ingestible device (10) comprises a first chamber (11) being enlargeable in space, comprising an inlet (6), and to be filled with the material to be sampled, a second chamber (12) comprising a solid block (2) and at least one release member (14) pressed against the solid block (2) to restrict the first chamber (11) from enlarging in space, wherein the solid block (2) is malleable when it is heated above a threshold temperature, and an actuating mechanism. In this context, the actuating mechanism is configured for, when triggered, heating the solid block (2) above the threshold temperature so that the at least one release member (14) is pushed into the solid block (2) allowing the first chamber (11) to enlarge and to collect the material to be sampled through the inlet (6).

## Description

### Technical field

The invention relates generally to an ingestible device and more specifically to an ingestible device for sampling material using a heat dependent actuating mechanism and a method for sampling material using such ingestible device.

### Technical background

Generally, to gather information and/or samples from the gastrointestinal (GI) tract, endoscopy is often performed. Endoscopes are basically long tubes that can be equipped with a camera, cutting tools, and hollow needles. The proximal and distal parts of the GI tract are reachable via endoscopy. Unfortunately, the endoscopic procedure is rather unpleasant for the patient. On top of that, the small intestine cannot be reached via this procedure. Therefore, the need for a device that enables investigation of the small intestine contents without disturbing the user is essential.

US 2021/0345904 A1 relates to an ingestible capsule device which collects fluid aspirates from locations within the body, locations such as the small intestine, and retains the fluid aspirates free from contamination as the capsule device is expelled from the body. Said capsule device employs a peristaltic pump fluid control with the capsule device, and a single semi-permeable bladder store collected fluid aspirate. Disadvantageously, especially due to the usage of said peristaltic pump and the rather high energy demand and space requirement associated therewith, said ingestible capsule device cannot provide enough energy and free space for sensors exemplarily measuring GI fluid in real time.

### Summary of the invention

The invention is set out in the appended set of claims.

It is an object of the present disclosure to provide an ingestible device, with enough energy and free space for sensors, exemplarily measuring GI fluid in real time especially due to an energy-saving, and thus also space-saving, actuating mechanism.

According to a first aspect of the present description, an ingestible device for sampling material in a gastrointestinal (GI) tract is provided. The ingestible device comprises a first chamber being enlargeable in space, comprising an inlet, and to be filled with the material to be sampled, a second chamber comprising a solid block and at least one release member pressed against the solid block to restrict the first chamber from enlarging in space, wherein the solid block is malleable when it is heated above a threshold temperature; and an actuating mechanism. In this context, the actuating mechanism is configured such that triggering the actuating mechanism leads to heating the solid block above the threshold temperature so that the at least one release member is pushed into the solid block allowing the first chamber to enlarge and to collect the material to be sampled through the inlet. Advantageously, it is easy to assemble this actuating mechanism in the ingestible device or the sampling pill. Also, the energy that is required to heat the solid block is within the actuation energy that a power source of the ingestible device can deliver.

Moreover, the whole actuating mechanism is very simple and consumes very little energy for actuation. Consequently, this allows for an energy-saving, and thus also space-saving, actuating mechanism, thereby enabling the provision of enough energy and free space for sensors, exemplarily measuring GI fluid in real time.

With respect to the actuating mechanism, it is noted that the actuating mechanism can manually be brought into its inactivated state after it has been activated or after the usage of the ingestible device, respectively. In this context, the actuating mechanism may typically be brought into its inactivated state not in situ.

In addition to this, it is noted that triggering the actuating mechanism may especially comprise actively triggering the actuating mechanism exemplarily in a remote manner.

As a further advantage, it is noted that the ingestible device described herein is suitable for applications including human or animal users.

According to an example implementation, the first chamber collects the material through the inlet due to an under pressure, wherein the inlet is especially sealed when the material is collected.

According to another example, further additionally or further alternatively, the ingestible device further comprises a third chamber being enlargeable in space, comprising a further inlet, and to be filled with further material to be sampled, a fourth chamber comprising a further solid block and at least one further release member pressed against the further solid block to restrict the third chamber from enlarging in space, wherein the further solid block is malleable when it is heated above the threshold temperature; and a further actuating mechanism. In this context, the further actuating mechanism is configured such that triggering the further actuating mechanism leads to heating the further solid block so that the at least one further release member is pushed into the further solid block allowing the third chamber to enlarge and to collect the material to be sampled through the further inlet. Advantageously, for instance, a second sample can be taken with the same ingestible device especially at a different location in the GI tract.

Further advantageously, according to an example, it is noted that the ingestible device can be used for simultaneously sampling the material at the first chamber and the third chamber. Also, it may be noted that the actuating mechanism and the further actuating mechanism may be triggered at different time intervals so that the first chamber and the third chamber may collect the sample at their respective time intervals.

According to a second example implementation, the first chamber and the second chamber are axisymmetric with respect to the third chamber and the fourth chamber. In addition to this or as an alternative, the actuating mechanism is axisymmetric with respect to the further actuating mechanism.

According to another example, further advantageously, additionally or alternatively, the first chamber may be mirror-symmetric with the third chamber and/or the second chamber may be mirror-symmetric with the fourth chamber, wherein the respective mirror plane may especially be a cross section in the center of the ingestible device. Additionally or alternatively, the two major segments or halves, respectively, of the ingestible device are basically identical.

According to a further example implementation, the actuating mechanism comprises: a spring being compressed if the actuating mechanism has not been triggered yet, a heating element operatively coupled to the solid block. In this context, the heating element is configured to heat the solid block if the actuating mechanism is triggered. It may be noted that the actuating mechanism may also be referred to as a heat dependent actuating mechanism.

Additionally, the spring is arranged with respect to the solid block and the at least one release member such that heating the solid block above the threshold temperature allows the at least one release member to be pushed into the solid block and leads to a decompression of the spring. Furthermore, the spring is arranged with respect to the first chamber such that the first chamber is enlarged in the case of the decompression of the spring.

According to a further example implementation, the solid block comprises an indentation within which the heating element is placed. Also, the heating element is coupled to an energy source to heat the solid block when the actuating mechanism is triggered.

Additionally, the at least one release member is pressed against the solid block in such a way that a portion of the at least one release member protrude from the second chamber to hold or restrict the first chamber from enlarging when the actuating mechanism is not triggered. Further, when the actuating mechanism is triggered, the heating element heats the solid block so that at least the portion of the at least one release member is pushed into the second chamber to release the first chamber to enlarge and to collect the sample material in the GI tract.

According to a further example implementation, the further actuating mechanism comprises a further spring being compressed if the further actuating mechanism has not been triggered yet, and a further heating element operatively coupled to the further solid block. In this context, the further heating element is configured to heat the further solid block if the further actuating mechanism is triggered. It may be noted that the further actuating mechanism may also be referred to as a heat dependent further actuating mechanism.

Additionally, the further spring is arranged with respect to the further solid block and the at least one further release member such that heating the further solid block allows the at least one further release member to be pushed into the further solid block and leads to a decompression of the further spring. Also, the further spring is arranged with respect to the third chamber such that the third chamber is enlarged in the case of the decompression of the further spring.

According to a further example implementation, the inlet comprises a valve, preferably a passive valve, more preferably a passive one-way valve, most preferably an umbrella valve. Additionally or alternatively, especially in the case of the presence of the further inlet, the further inlet comprises a further valve, preferably a further passive valve, more preferably a further passive one-way valve, most preferably a further umbrella valve. Advantageously, the umbrella valve and/or the further umbrella valve may be made of silicon rubber.

According to a further example implementation, the first chamber comprises a removable portion, preferably a screwable cap, for access to the internal space of the first chamber. Additionally or alternatively, especially in the case of the presence of the third chamber, the third chamber comprises a further removable portion, preferably a further screwable cap, for access to the internal space of the third chamber. Advantageously, for instance, this allows for an effortless sample retrieval.

According to a further example implementation, the material comprises or is gastrointestinal content. Further additionally or further alternatively, especially in the case of the presence of the further material, the further material comprises or is further gastrointestinal content.

According to a further example implementation, at least a part of the inner space of the first chamber comprises a stabilizing substance, preferably a quencher, especially for preventing the material from additional chemical reactions. Additionally or alternatively, especially in the case of the presence of the third chamber, at least a part of the inner space of the third chamber comprises a further stabilizing substance, preferably a further quencher, especially for preventing the further material from additional chemical reactions. Advantageously, for instance, digestion and fermentation of the sampled content can be prevented in a particularly efficient manner.

According to a further example implementation, the ingestible device further comprises a body portion especially providing a hollow inner space being preferably sealed against the environment of the body portion. In this context, the first chamber and the second chamber are attached to the body portion. Additionally or alternatively, especially in the case of the presence of the third chamber and the fourth chamber, the third chamber and the fourth chamber are attached to the body portion preferably in a symmetric manner to the first chamber and the second chamber. Advantageously, for example, sample contamination can be prevented in a cost-efficient manner.

According to a further example implementation, the body portion, especially the hollow inner space of the body portion, comprises at least one of an energy source for supplying electric power to the actuating mechanism and/or in the case of the presence of the further actuating mechanism, for supplying electric power to the further actuating mechanism. Further, the body portion comprises a remote trigger unit, being especially supplied the electric power by the energy source, and configured to receive at least one remote trigger signal in order to trigger the actuating mechanism and/or in the case of the presence of the further actuating mechanism, to trigger the further actuating mechanism. In addition, the body portion comprises a data recording unit for recording data, preferably environment data and/or localization data, during usage of the ingestible device. Furthermore, the body portion comprises a wireless data transmission unit for wirelessly transmitting data, preferably environment data and/or localization data, more preferably environment data and/or localization data in real time, during usage of the ingestible device. Additionally or alternatively, the body portion, especially not the hollow inner space of the body portion, comprises at least one sensor, preferably a pH-value sensor. Advantageously, for instance, there is plenty of room for the energy source, electronics such as communication, preferably wireless communication, and at least one antenna, and sensors.

According to a further example implementation, the ingestible device has the shape of a pill or a cylinder. Additionally or alternatively, the length of the ingestible device is lower than 31 millimeters preferably after the actuating mechanism has been triggered and/or in the case of the presence of the further actuating mechanism, the further actuating mechanism has been triggered. Further additionally or further alternatively, the diameter of the ingestible device is lower than 10 millimeters, preferably after the actuating mechanism has been triggered and/or in the case of the presence of the further actuating mechanism, the further actuating mechanism has been triggered. Advantageously, for example, the ingestible device is small enough in size that it can easily be swallowed.

According to a second aspect of the present description, a method for sampling material using an ingestible device according to any of the implementation forms described herein is provided. The method comprises the step of triggering the actuating mechanism by a first remote trigger signal and/or in the case of the presence of the further actuating mechanism, triggering the further actuating mechanism by transmitting a second remote trigger signal.

### Brief description of the drawings

Exemplary embodiments of the ingestible device and method for sampling material are now further explained with respect to the drawings by way of example only, and not for limitation. In the drawings:
- Fig. 1A: shows an example embodiment of an ingestible device in an inactivated state according to the present description;
- Fig. 1B: shows an example embodiment of the ingestible device according to Fig. 1A in an activated state;
- Fig. 2: shows an example embodiment of the ingestible device according to Fig. 1A or Fig. 1B, respectively, as a blow-up drawing;
- Fig. 3: shows the illustration of a portion of the ingestible device of Fig. 1A or Fig. 1B, as a blow-up drawing;
- Fig. 4A-4C: shows an example embodiment of a solid block with a heating element in an inactivated state according to the present description;
- Fig. 5A-5C: shows an example embodiment of a solid block with a heating element in an activated state according to the present description;
- Fig. 6: shows a block diagram of a body portion, a heating element, a solid block, in accordance with aspects of the present description; and
- Fig. 7: shows a flow chart of an example of the method for sampling material using an ingestible device according to the present description.

### Detailed description

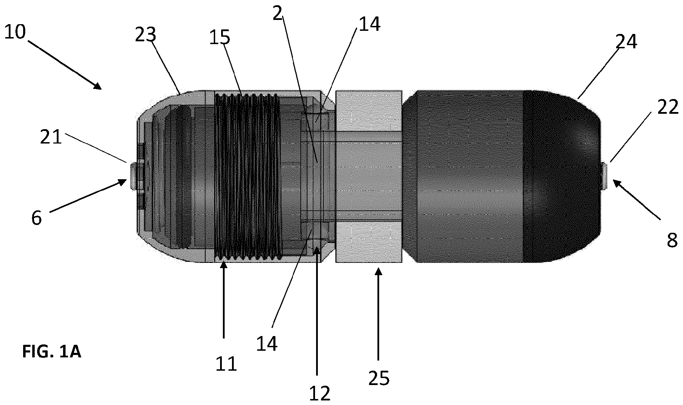

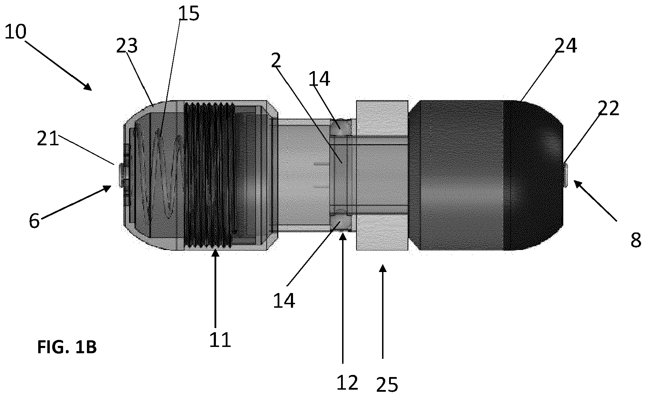

With respect to Figs. 1A and 1B, an exemplary embodiment of an ingestible device 10 for simultaneously sampling material is illustrated. Fig. 1A depicts the ingestible device 10 in an inactivated state, while Fig. 1B depicts the ingestible device 10 in an activated state. It may be noted that the inactivated state may be referred to as a state where the actuating mechanism and/or the further actuating mechanism are not triggered, and the solid block and/or the further solid block are not malleable. In one example, the solid block being malleable is referred to as a state where it does not hold its shape like a solid but does not flow like a liquid. Also, when the solid block is malleable, the release member may glide into the solid block. The solid block may be made of material such as polycaprolactone (PCL) or any other biocompatible material with a low melting temperature. It may be noted that the solid block becomes malleable when it is heated above a threshold temperature for example, in a range from 42 degrees centigrade to 60 degrees centigrade. It may be noted that the low melting temperature is less than or equal to the threshold temperature. The solid block may be of any shape, and it is not limited to the shape shown in Figs. 1A to 5C. In one example, the solid block may also be referred to as a block having hollow core at the center and a solid frame around the hollow core.

According to Fig. 1A, the ingestible device 10 comprises a first chamber 11 being enlargeable in space, comprising an inlet 6, and to be filled with the material to be sampled. In one example, the material may be referred to as gastrointestinal (GI) content that is collected when the ingestible device 10 passes through the GI tract of a body such as, human body or animal body. The space may be referred to as the volume of the chamber 11 that is increased as depicted in Fig. 1B.

Further, the ingestible device 10 comprises a second chamber 12 that is concentrically positioned with respect to the first chamber 11. Also, the second chamber 12 is tightly sealed to the first chamber 11 in such a way that it allows the first chamber 11 to enlarge when the actuating mechanism is triggered.

In accordance with Figs. 1A and 1B, the second chamber 12 comprises a solid block 2 that is positioned within a hollow portion of the second chamber 12. Also, the second chamber 12 comprises one or more release members 14 that are pressed against the solid block 2 to restrict the first chamber 11 from enlarging in space. It may be noted that the second chamber 12 may comprise any number of release members, and it is not limited to the number shown in Figs. 1A and 1B. Also, the release member 14 may be of any shape and size that is capable of holding the first chamber 11 when the ingestible device 10 is in an inactivated state, and further releasing the first chamber 11 when the ingestible device 10 is activated. In one example, the release member 14 may be a ball shaped member as shown in Figs. 1A to 5C. In another example, the release member 14 may be an elongated structure with a semispherical tip or a tapered tip that helps to restrict the first chamber 11 from enlarging when the device 10 is in the inactivated state.

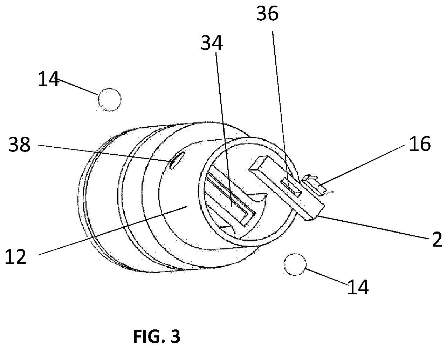

In the embodiment of Figs. 1A and 1B, the second chamber 12 comprises two apertures on two opposite sides of the surface (see Fig. 3). Further, each release member 14 is aligned with the corresponding aperture in such a way that a portion of the release member 14 protrudes outside the aperture of the second chamber 12 to hold or restrict the first chamber 11 from enlarging. Also, the remaining portion of the release member 14 is within the second chamber 12 and pressed against the solid block 2. The space may be referred to as the volume of the chamber that is increased as depicted in Fig. 1B.

In addition, the solid block 2 is malleable when it is heated above a threshold temperature. In one example, the threshold temperature is in a range from about 42 degrees centigrade to about 60 degrees centigrade. Once the solid block 2 becomes malleable, the release members 14 are pushed into the malleable solid block 2. In particular, a portion of the first chamber 11 that is on top of the protruded release members 14 may exert force on the release members 14. In one example, the portion of the first chamber 11 may be a rim like structure of the first chamber 11 that locks with the protruded release members 14. Consequently, when the solid block 2 is malleable, the release members 14 are pushed into the solid block 2.

As the portion of the release member 14 that is outside the aperture of the second chamber 12 is pushed into the second chamber 12, the first chamber 11 is released for enlarging. In one example, a rim like structure of the first chamber 11 gets unlocked due to the downward movement of the release member 14. As a result, the first chamber 11 enlarges due to decompression of the spring 15. Also, the released first chamber 11 collects the sample material while it is enlarged into space.

In addition, the ingestible device 10 comprises an actuating mechanism. In this context, the actuating mechanism is configured such that triggering the actuating mechanism leads to an enlargement of the first chamber 11. More specifically, when the actuating mechanism is triggered, the solid block 2 is heated above the threshold temperature. As a result, the solid block 2 becomes malleable, and the release members 12 are pushed into the solid block 2. Consequently, the first chamber 11 is released to enlarge and to collect the material to be sampled through the inlet 6 especially due to an under pressure.

Similar to the first chamber 11 and the second chamber 12, the ingestible device further comprises a third chamber and a fourth chamber on the other side of the body portion 25. The arrangement of the third chamber and the fourth chamber is symmetric and mirror view of the first chamber 11 and the second chamber 12. Also, the third chamber and the fourth chamber may function similar to the first chamber 11 and the second chamber 12, respectively. The third and fourth chambers may not be seen clearly in Figs. 1A and 1B. For ease of understanding of the embodiments of the description, only the left portion of the ingestible device 10 is depicted as transparent to show the internal components and their movements, in Figs. 1A and 1B.

Furthermore, the third chamber being enlargeable in space, comprising a further inlet 8, and to be filled with further material to be sampled. The further material may be similar to the above-mentioned material that is referred to as gastrointestinal (GI) content that is collected when the ingestible device 10 passes through the GI tract of the body.

In accordance with Figs. 1A and 1B, the fourth chamber is concentrically positioned with respect to the third chamber. Further, the fourth chamber comprises a further solid block that is positioned within a hollow portion of the fourth chamber. Similar to the release members in the second chamber 12, the fourth chamber also comprises release members that are pressed against the solid block to restrict the third chamber from enlarging in space.

In addition, the further solid block is malleable when it is heated above a threshold temperature. Once the further solid block is malleable, the respective release members are pushed into the malleable further solid block. As the portion of the release members that is outside the aperture of the fourth chamber is pushed into the fourth chamber, the third chamber is released for enlarging. Also, the released third chamber collects the further sample material while it is enlarged into space.

In addition, the ingestible device 10 comprises a further actuating mechanism. In this context, the further actuating mechanism is configured such that triggering the further actuating mechanism leads to heating the further solid block so that the at least one further release member is pushed into the further solid block allowing the third chamber to enlarge and to collect the further material to be sampled through the further inlet especially due to an under pressure.

Although Figs 1A and 1B show two chambers for sampling material, it may be noted that, in one embodiment, the ingestible device 10 may include only one chamber for sampling material. For example, the ingestible device 10 may include only the first chamber 11 and the second chamber 12, without the third chamber and the fourth chamber. Also, the actuating mechanism may be activated/triggered independent of triggering the further actuating mechanism.

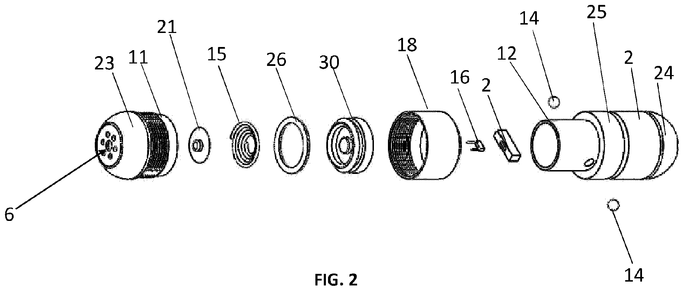

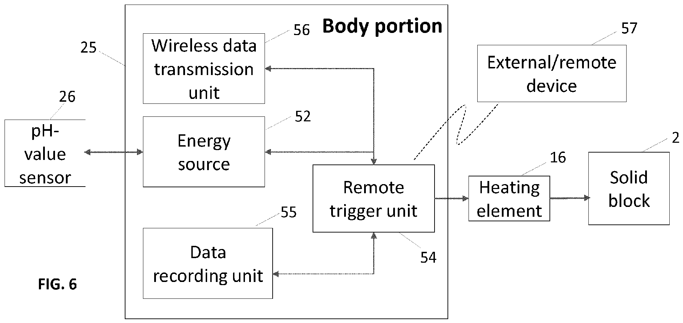

With respect to the above-mentioned actuating mechanism, it is noted that the actuating mechanism comprises a spring 15 and a heating element 16 (see Fig. 2). The heating element 16 may be a standard RC0402 resistor or a nickel chromium wire. The spring 15 is compressed if the actuating mechanism has not been triggered yet. Further, the heating element 16 is operatively coupled to the solid block 2. Also, the heating element 16 is configured to heat the solid block 2 if the actuating mechanism is triggered. In particular, the heating element 16 being conductively connected to an energy source 52 if the actuating mechanism is triggered. This set-up may be seen in Fig. 6. In this context, the heating element 16 is configured such that connecting the heating element 16 to the energy source 52 leads to heating the solid block 2. Once the solid block 2 is heated above the threshold temperature, it becomes malleable and allows the release ball members 14 to be pushed into the solid block 2. In one example, the energy source 52 may provide a power in a range from about 0.1 mWh to 0.5 mWh to the heating element 16 so that the solid block 2 is heated above the threshold temperature.

In addition to this, the spring 15 is arranged with respect to the solid block 2 and the at least one release member 14 such that heating the solid block 2 above the threshold temperature allows the at least one release member 14 to be pushed into the solid block 2 and leads to a decompression of the spring 15. Also, as shown in Fig. 1B, the spring 15 is arranged with respect to the first chamber 11 such that the first chamber 11 is enlarged in the case of the decompression of the spring 15.

In accordance with Figs. 1A and 1B, with respect to the further actuating mechanism, it is noted that the further actuating mechanism comprises a further spring and a further heating element. It may be noted that the further heating element may be similar to the heating element 16 shown in Figs. 2 and 3. The further spring is compressed if the further actuating mechanism has not been triggered yet.

Further, the further heating element is operatively coupled to the further solid block. Also, the further heating element is configured to heat the further solid block if the further actuating mechanism is triggered. In particular, the further heating element, being conductively connected to a further energy source if the further actuating mechanism is triggered. In this context, the further spring is arranged with respect to the further solid block and the at least one further release member such that heating the further solid block allows the at least one further release member to be pushed into the further solid block and leads to a decompression of the further spring. Also, the further spring is arranged with respect to the third chamber such that the third chamber is enlarged in the case of the decompression of the further spring.

With respect to the above-mentioned inlet 6, it is noted that the inlet 6 comprises a valve, preferably a passive valve, more preferably a passive one-way valve, most preferably an umbrella valve such as the umbrella valve 21 of Fig. 2. With respect to the above-mentioned further inlet 8, it is noted that the further inlet comprises a further valve, preferably a further passive valve, more preferably a further passive one-way valve, most preferably a further umbrella valve such as the further umbrella valve. It may be noted that the valve 21 or the further valve allows flow of material from outside the ingestible device 10 into the chamber (first or third chamber) but, blocks or ceases the material to flow out of the chamber (first chamber or third chamber).

In addition, at least a part of the inner space of the first chamber 11 comprises a stabilizing substance, preferably a quencher, especially for preventing the material from additional chemical reactions. In a similar manner, at least a part of the inner space of the third chamber comprises a further stabilizing substance, preferably a quencher, especially for preventing the further material from additional chemical reactions. Advantageously, for instance, digestion and fermentation of the sampled content can be prevented in a particularly efficient manner by using the stabilizing substance or the further stabilizing substance.

Furthermore, as can be seen from Figs. 1A and 1B or Fig. 2, respectively, the first chamber 11 comprises a removable portion, exemplarily a screwable cap 23, for access to the internal space of the first chamber 11. Moreover, the third chamber comprises a further removable portion, exemplarily a further screwable cap 24, for access to the internal space of the third chamber.

In accordance with Fig. 1A, the ingestible device 10 further comprises a body portion 25 especially providing a hollow inner space being preferably sealed against the environment of the body portion 25. In this context, the first chamber 11 and the second chamber 12 are attached to the body portion 25, and the third chamber and the fourth chamber are attached to the body portion 25 exemplarily in a symmetric manner to the first chamber 11 and the second chamber 12.

It might be particularly advantageous if the body portion 25, especially the hollow inner space of the body portion 25, comprises at least one of an energy source for supplying electric power to the actuating mechanism and the further actuating mechanism. Also, the body portion 25 comprises a remote trigger unit, being especially supplied the electric power by the energy source, and configured to receive at least one remote trigger signal in order to trigger the actuating mechanism and the further actuating mechanism.

Further, the body portion 25 comprises a data recording unit for recording data, preferably environment data and/or localization data, during usage of the ingestible device 10. In one example, the environment data could be pH and temperature. This data is also used to roughly determine the position of the pill in the GI tract. Other environmental data could be inflammation markers. It may be noted that the body portion 25 is described in greater detail with respect to Fig. 6.

In addition to this or as an alternative, the body portion 25, especially not the hollow inner space of the body portion 25, may comprise at least one sensor 26, preferably a pH-value sensor. In one example, the pH-value sensor may be used to determine or measure the pH-value at one or more locations in the GI tract. It is further noted that the ingestible device 10 has the shape of a pill or a cylinder.

As it can be seen from Fig. 1A showing the inactivated state of the ingestible device 10 and Fig. 1B illustrating the activated state of the same, the diameter of the ingestible device 10 does not change after activation, whereas the length of the ingestible device 10 increases due to activation. In this context, the length of the ingestible device 10 is lower than 31 millimeters, exemplarily after the actuating mechanism has been triggered and/or the further actuating mechanism has been triggered. Also, the diameter of the ingestible device 10 is lower than 10 millimeters.

With respect to the inactivated state of the ingestible device 10 according to Fig. 1A, it is noted that in this state, neither the actuation mechanism nor the further actuation mechanism have been triggered yet. Accordingly, both the spring 15 and the further spring are still compressed. With respect to the activated state of the ingestible device 10 according to Fig. 1B, it is further noted that in this state, at least one, exemplarily both, of the actuation mechanism and the further actuation mechanism has been triggered, which leads to a decompression of the respective one of the springs 15 and the further spring.

Again, with respect to Fig. 2A, it is noted that the screwable cap 23, the umbrella valve 21, the spring 15, the further screwable cap 24, and the body portion 25 have already been explained in the context of Fig. 1A or Fig. 1B, respectively.

Furthermore, a connector 18 is provided between the first chamber 11 and the second chamber 12. In particular, when the actuating mechanism is not triggered yet, the first chamber 11 is concentrically positioned over the connector 18 and held close to the second chamber 12. The protruded release members 14 prevent or restrict the first chamber 11 from enlarging. Further, when the actuating mechanism is triggered, the protruded release members 14 are pushed into the solid block 2, thereby causing the first chamber 11 to enlarge by sliding over the surface of the connector 18.

In addition, the spring 15, the O-ring 26, the umbrella valve 21, and the sealer 30 are provided between the first chamber 11 and the connector 18 to provide an exerting force on the first chamber to enlarge in space. More specifically, the spring 15, the O-ring 26, and the sealer 30 are positioned within the hollow portion of the connector 18. Also, the spring 15 is compressed when the actuating mechanism is not triggered to provide a spring force or a restoring force on the first chamber 11.

Furthermore, in view of Fig. 2, it is noted that the solid block 2 has already been explained in the context of Fig. 1A or Fig. 1B. Furthermore, positioning of the solid block 2 in the second chamber 12 and other internal components such as, a heating element 16 and the release members 14 will be explained in greater detail with respect to Fig. 3.

Fig. 3 shows the blown-up view of a solid block 2, a heating element 16, and release members 14 in the second chamber 12. It can be seen that the second chamber 12 has a slot 34 within which the solid block 2 is placed. Also, the slot 34 is designed in such a way that the solid block 2 is held firmly irrespective of the movement of the ingestible device 10 in the GI tract. It may be noted that the slot 34 is provided within a hollow portion of the second chamber 12. Further, the solid block 2 has an indentation 36 at the bottom as shown in Fig. 3. The indentation 36 allows the heating element 16 to be positioned within the solid block 2 and to be in direct contact with the solid block 2. In addition, the heating element 16 is electrically coupled to an energy source (not shown) to heat the solid block 2 when the actuating mechanism is triggered.

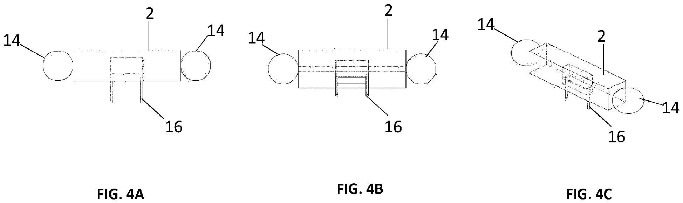

As depicted in Fig. 3, the second chamber 12 has apertures 38 on the surface of the second chamber 12. The apertures 38 are on two opposite sides of the second chamber 12. Further, these apertures 38 are aligned with the sides of the solid block 2 inserted into the slot 34 of the second chamber 12. Also, as depicted in Figs. 4A-4C, the release members 14 are positioned on the sides of the solid block 2 in such a way that a portion of the release members 14 protrude out of the second chamber 12 via the aperture 38. This protruded portion of the release members 14 holds the first chamber 11 and restricts it from enlarging when the actuating mechanism is not triggered yet. More specifically, the compressed spring in the first chamber 11 may exert force on the walls of the first chamber 11 in a direction opposite to the body portion 25. But the release members 14 that protrude outside the second chamber 12 may hold the first chamber 11 and restricts it from enlarging in space.

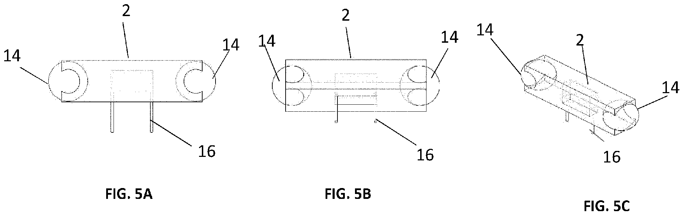

Furthermore, when the solid block 2 is heated above the threshold temperature, the solid block 2 becomes malleable and these release members 14 move or pushed into the malleable solid block 2 as depicted in Figs. 5A-5C. As a result, the protruded portion of the solid block 2 is also pushed into the second chamber 12, causing the first chamber 11 to expand or enlarge due to the force exerted by the compressed spring 15. In particular, the spring 15 decompresses and the first chamber 11 enlarges to collect the sample material through the inlet 6. In one embodiment, the solid block 2 and the heating element 16 may be molded as a single unitary structure.

With respect to the body portion 25, it is noted that said body portion, especially its hollow inner space, may comprise one or more electronics modules as depicted in Fig. 6. It might be particularly advantageous if said electronic modules comprise at least an energy source 52, a remote trigger unit 54, a data recording unit 55, and a wireless data transmission unit 56. The energy source 52 is configured to supply electric power to the actuating mechanism and the further actuating mechanism. Also, the energy source 52 may supply electric power to the remote trigger unit 54, the data recording unit 55, and the wireless data transmission unit 56. Further, the remote trigger unit 54 is configured to receive at least one remote trigger signal in order to trigger the actuating mechanism and the further actuating mechanism. In one example, the remote trigger unit 54 may receive the remote trigger signal from an external or remote device 57 as shown in Fig. 6.

In this context, if the actuating mechanism is triggered, the energy source 52 is coupled to the heating element 16 to heat the solid block 2. In one example, if the heating element 16 is coupled to the energy source 52, the heating element 16 heats up or the temperature increases due to joule heating. As a consequence, the solid block 2 that is coupled to the heating element 16 may also heat up.

Furthermore, the data recording unit 55 is configured to record data, preferably environment data and/or localization data, during usage of the ingestible device 10. The wireless data transmission unit 56 is configured to wirelessly transmit data, preferably environment data and/or localization data, more preferably environment data and/or localization data in real time, during usage of the ingestible device 10. In one embodiment, the wireless data transmission unit 56 may also be configured to wirelessly receive the remote trigger signal from the external device 57 and transmit the remote trigger signal to the remote trigger unit 54. In addition, the said electronics module may comprise at least one sensor 26, preferably a pH-value sensor for sensing pH-value at a desired location in the GI tract.

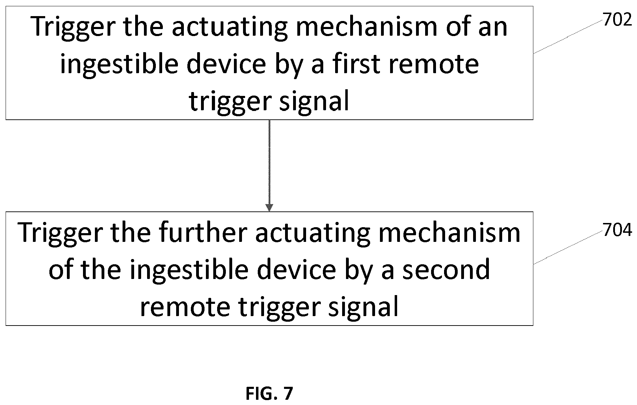

Before explaining the final Fig. 7 showing an exemplary embodiment of the method for sampling material using an ingestible device, the ingestible device according to the present description and its advantages are summarized in the following. The exemplary embodiment of the inventive ingestible device 10 described above can be seen as a remotely activatable dual-sampling pill, which especially consists of only a few elements. Thus, manufacturing such an ingestible device is advantageously very cheap.

The solid block 2 and the heating element 16 are strong enough to withstand some pressure. Therefore, it can function as a strut to keep the outer shell of the ingestible device 10 fixed in the inactivated state. Due to the malleable nature of the solid block 2, it can function as part of the actuating mechanism. In addition, the actuating mechanism contains fewer complex mechanical parts and a simpler design compared to US 2021/0345904 A1. Therefore, the costs of manufacturing and assembly can be much lower. Also, the reliability of the actuation mechanism can be much better.

The cap lid, such as the screwable cap 23 and the further screwable cap 24 of Figs. 1A and 1B, can be screwed on the cap section. Furthermore, the cap lid contains a passive umbrella valve such as the umbrella valve 21 and the further umbrella valve according to Fig. 1A and 1B. This passive umbrella valve enables one-directional flow, enabling sampling and closing of the top of the sample chamber such as the first chamber 11 and the third chamber of Fig. 1A and 1B. The bottom of the sample chamber is sealed off by an O-ring, such as the O-rings 26 and a sealer 30 according to Fig. 2.

Advantageously, the screwable cap lid enables easy access to the sample chamber. This can be very helpful for effortless injecting a quencher as well as simple sample removal. The overall size of the remotely activable dual-sampling pill in the inactivated state is not exceeding 31 millimeters in length and/or 10 millimeters in diameter, which advantageously makes the pill relatively easy to swallow. Advantageously, the hollow electronics module or the hollow body portion, respectively, has enough space inside to implement the power/energy source, the sensors, and further electronics such as communication, preferably wireless communication, and at least one antenna.

Moreover, as a further advantage, the middle section of the body portion, such as the body portion 25 of Fig. 1A, is directly exposed to the gastrointestinal (GI) fluid.

Accordingly, sensors implemented in this area of the pill can therefore measure GI fluid in real time. These measurements contribute to the place determination of the pill and give insight into the user's health.

Further advantageously, the power source or energy source, respectively, can generate more than enough current to activate the actuating mechanism. The actuating mechanism especially causes heating of the heating element. Especially due to this heating, the solid block is heated above the threshold temperature, allowing a vertical or lateral movement of the compression spring such as the spring 15.

This lateral movement enlarges the volume of the sample chamber, creating an under pressure that will be released by taking in GI fluid from the GI tract at the desired location.

As it can generally be seen, the remotely activable dual-sampling pill especially consists out of three major parts that slide over each other, allowing for an expansion of the device.

Once the device reaches the activated state, it hovers around dimensions not exceeding 31 millimeters in length and/or 10 millimeters in diameter. As already mentioned above, the remotely activable dual-sampling pill comprises only a few elements and most or even all of them are very easy to fabricate, for example via 3D printing among other methods. The sliding mechanism is a reliable way to achieve sampling, while diminishing the chance of failure. Moreover, the remotely activable dual-sampling pill in its entirety preferably is nonmagnetic, making it MRI (magnetic resonance imaging) compatible.

Further advantageously, the ingestible device has two actuating mechanisms, one for each sampling chamber. Each of these mechanisms may especially comprise a heating element that after heating causes the solid block to be malleable. As the solid block is malleable, the spring decompresses which in-turn enables the capsule module to freely move in a lateral direction. The lateral movement is achieved by decompression of the spring.

It is particularly advantageous that this actuating mechanism requires very little current as well as a sufficiently low voltage, and therefore avoids having to make use of large batteries making it extremely suitable for the remotely activatable dual-sampling pill. It is further noted again that sampling and delivery are enabled by the previously discussed lateral movement of the capsule caused by the release of the compression spring. The lateral movement of the capsule induces an expansion of the sample chamber forming an under pressure inside the sample chamber that can only be released by taking in a sample.

As already discussed above, the inventive ingestible device advantageously provides closed off compartments because the sample chambers are sealed off by an umbrella valve on one end and by an O-ring and a sealer on the other end. Further advantageously, both components are very cheap and easily obtainable. As a further advantage, the inventive ingestible device allows for easy sample removability because the capsule modules have a screwable cap that allows for direct sample removal. Accordingly, the chance of sample spillage is made non-existent.

Furthermore, as already discussed above, the ingestible device according to the present description provides possible space for sensors because the electronics module of the remotely activable dual-sampling pill is large enough to contain sensors. The sensors can advantageously determine time and position to enable sampling at the correct location in the GI tract. Moreover, this also allows for continuous measurements of GI fluid.

The major advantages of the remotely activatable dual-sampling pill or the inventive ingestible device, respectively, can be summarized in a nutshell as follows:
1. The device allows for noninvasive active gastrointestinal fluid sampling.
2. The device is simple to fabricate exemplarily via 3D printing. Other parts are easily obtainable.
3. The screwable cap lids enable effortless sample retrieval.
4. The spacious interior of the electronics module or the body part leaves plenty of room for the energy source, electronics, and sensors. The implemented sensors that measure GI fluid in real time contribute to the place determination of the pill and give insight into the user's health.
5. Due to overall pill size, in the inactivated state, not exceeding the already discussed size, the administration of the pill is comfortable for the user.
6. The remotely activatable dual-sampling pill is MRI compatible.
7. The remotely activatable dual-sampling pill comprises a build in localization component.
8. There is no sample contamination due to closed off sample chambers by passive umbrella valves and O-rings.
9. The remotely activatable dual-sampling pill is very easy to assemble.
10. Due to overall pill size, in the activated state, not exceeding the size as discussed above, the chance of pill retention is strongly reduced.

Finally, Fig. 7 shows a flow chart of an embodiment of a method for sampling material using an ingestible device according to the present description such as the ingestible device 10 as explained above. In step 702, the actuating mechanism of the ingestible device is triggered by a first remote trigger signal. The first remote trigger signal may be a trigger signal transmitted from the external device 57 to the remote trigger unit 54 as depicted in FIG. 6. In addition to this or as an alternative, in step 704, the further actuating mechanism of the ingestible device is triggered by transmitting a second remote trigger signal. The second remote trigger signal may be a trigger signal transmitted from the external device 57 to the remote trigger unit 54 as depicted in FIG. 6. The second remote trigger signal is used to activate the further actuating mechanism so that the material is collected in the third chamber and the liquid stored in the fourth chamber is released to the environment of the ingestible device 10.

While various embodiments of the present invention have been described above, it should be understood that they have been presented by way of example only, and not limitation. Numerous changes to the disclosed embodiments can be made in accordance with the disclosure herein without departing from the spirit or scope of the invention. Thus, the breadth and scope of the present invention should not be limited by any of the above described embodiments. Rather, the scope of the invention should be defined in accordance with the following claims and their equivalents.

Although the invention has been illustrated and described with respect to one or more implementations, equivalent alterations and modifications will occur to others skilled in the art upon the reading and understanding of this specification and the annexed drawings. In addition, while a particular feature of the invention may have been disclosed with respect to only one of several implementations, such feature may be combined with one or more other features of the other implementations as may be desired and advantageous for any given or particular application.

## Claims

1. An ingestible device (10) for sampling material in a gastrointestinal tract, the ingestible device (10) comprising:
a first chamber (11) being enlargeable in space, comprising an inlet (6), and to be filled with a material to be sampled;
a second chamber (12) comprising a solid block (2) and at least one release member (14) pressed against the solid block to restrict the first chamber (11) from enlarging in space, wherein the solid block is malleable when it is heated above a threshold temperature; and
an actuating mechanism configured for, when triggered, heating the solid block above the threshold temperature so that the at least one release member is pushed into the solid block allowing the first chamber (11) to enlarge and to collect the material to be sampled through the inlet (6).

2. The ingestible device (10) according to claim 1, wherein the actuating mechanism comprises:
a spring (15) configured to be compressed if the actuating mechanism is not triggered, and configured to decompress if the actuating mechanism is triggered, and
a heating element (16) operatively coupled to the solid block (2), and wherein the heating element (16) is configured to heat the solid block (2) if the actuating mechanism is triggered.

3. The ingestible device (10) according to claim 2, wherein the spring (15) is arranged with respect to the solid block (2) and the at least one release member (14) such that heating the solid block (2) above the threshold temperature allows the at least one release member (14) to be pushed into the solid block (2) and leads to a decompression of the spring (15), wherein the spring (15) is arranged with respect to the first chamber (11) such that the first chamber (11) is enlarged in the case of the decompression of the spring (15).

4. The ingestible device (10) according to any of the preceding claims, wherein the solid block (2) comprises an indentation within which the heating element (16) is placed, and wherein the heating element (16) is coupled to an energy source to heat the solid block (2) when the actuating mechanism is triggered.

5. The ingestible device (10) according to any of the preceding claims, wherein the at least one release member (14) is positioned against the solid block (2) in such a way that a portion of the at least one release member (14) protrude from a corresponding aperture (38) of the second chamber (12) to hold the first chamber (11) from enlarging when the actuating mechanism is not triggered.

6. The ingestible device (10) according to any of the preceding claims, wherein, when the actuating mechanism is triggered, the heating element (16) heats the solid block (2) so that a portion of the at least one release member (14) is pushed into the second chamber (12) to allow the first chamber (11) to enlarge.

7. The ingestible device (10) according to any of the preceding claims, wherein the first chamber (11) collects the material through the inlet (6), and wherein the inlet (6) is especially sealed when the material is collected.

8. The ingestible device (10) according to any of the preceding claims, wherein the inlet (6) comprises a valve (21), preferably a passive valve, more preferably a passive one-way valve, most preferably an umbrella valve.

9. The ingestible device (10) according to any of the preceding claims, wherein the first chamber (11) comprises a removable portion, preferably a screwable cap (23), for access to the internal space of the first chamber (11).

10. The ingestible device (10) according to any of the preceding claims, wherein at least a part of the inner space of the first chamber (11) comprises a stabilizing substance, preferably a quencher, especially for preventing the sampled material from additional chemical reactions.

11. The ingestible device (10) according to any of the preceding claims, wherein the solid block (2) comprises polycaprolactone (PCL) or any other biocompatible material with a low melting temperature.

12. The ingestible device (10) according to any of the preceding claims, wherein the threshold temperature of the solid block (2) is in a range from 42 degrees centigrade to 60 degrees centigrade.

13. The ingestible device (10) according to any of the preceding claims, wherein the ingestible device further comprises a body portion (25) especially providing a hollow inner space being preferably sealed against the environment of the body portion (25), and wherein the first chamber (11) and the second chamber (12) are attached to the body portion (25).

14. The ingestible device (10) according to claim 13, wherein the body portion (25), especially the hollow inner space of the body portion, comprises at least one of:
an energy source (52) for supplying electric power to the actuating mechanism,
a remote trigger unit (54), being especially supplied the electric power by the energy source (52), and configured to receive at least one remote trigger signal in order to trigger the actuating mechanism,
a data recording unit (55) for recording data, preferably environment data and/or localization data, during usage of the ingestible device (10),
a wireless data transmission unit (56) for wirelessly transmitting data, preferably environment data and/or localization data, more preferably environment data and/or localization data in real time, during usage of the ingestible device (10), and/or wherein the body portion (25), especially not the hollow inner space of the body portion (25), comprises at least one sensor, preferably a pH-value sensor.

15. The ingestible device (10) according to any of the preceding claims, wherein the ingestible device (10) has the shape of a pill or a cylinder, and/or wherein the length of the ingestible device (10) is lower than 31 millimeters, preferably after the actuating mechanism has been triggered, and/or wherein the diameter of the ingestible device is lower than 10 millimeters.
